Europäisches Patentamt

European Patent Office

Office européen des brevets

(11) Publication number: **0 184 595**
A1

# EUROPEAN PATENT APPLICATION

(21) Application number: 84115454.5

(22) Date of filing: **14.12.84**

(51) Int. Cl.⁴: **C 07 D 209/48**

(43) Date of publication of application: **18.06.86**
**Bulletin 86/25**

(71) Applicant: **GENERAL ELECTRIC COMPANY, 1 River Road, Schenectady New York 12305 (US)**

(72) Inventor: **Cawse, James Norman, 132 Kittredge Road, Pittsfield Massachusetts 01201 (US)**

(84) Designated Contracting States: **DE FR GB IT NL SE**

(74) Representative: **Schüler, Horst, Dr. et al, Kaiserstrasse 41, D-6000 Frankfurt/Main 1 (DE)**

(54) **Process for the synthesis of N-alkylphthalimide.**

(57) High purity N-methylphthalimide is made directly from molten phthalic anhydride and gaseous monomethylamine in a continuous reactor.

## PROCESS FOR THE SYNTHESIS
## OF N-ALKYLPHTHALIMIDE

### BACKGROUND OF THE INVENTION

The compounds known as N-alkylphthalimides are used as intermediates in the production of nitro N-alkylphthalimides. These nitro N-alkylphthalimides are starting reactants for making a number of different organic dianhydrides and polyimides as shown by Heath et al., United States Patent No. 3,879,428.

Prior art processes for making N-alkylphthalimides such as N-methylphthalimide have been based on the reaction of phthalic anhydride with aqueous alkylamine. The alkylphthalimide is recovered by distillation of the reaction product. This method is objectionable because of the need to eliminate water in the recovery step.

The use of melt reaction conditions has resulted in the production of byproducts which are difficult to remove. These byproducts include N,N-dialkylphthalimides which interfere with reactions used to convert the N-alkylphthalimides into nitration products that are used to make organic dianhydrides and polyimides. A melt reaction for the production of N-alkylphthalimides is described in United States Patent No. 4,020,089 and is carried out in a hot tube reactor packed with a static bed of inert particles. The hot tube process is difficult to control in a large scale process because of the high heat of reaction involved in the formation of N-alkylimides such as N-methylphthalimide. Hot spots of up to 220°C. are typically recorded in this process. The exotherm from the making of N-methylphthalimide is approximately 39 kcal/g-mole. The yields for the process are described in Example 2 of United States Patent No. 4,020,-089 as being in the order of 72%.

Spring et al., J. Chem. Soc. 1945 pp. 625-628, describe processes wherein various reactions of N-alkylphthalimides and N,N'-dialkylphthalimides are used to make various derivatives. The heating of N,N'-dimethylphthalimide is described as a method of making N-methylphthalimide in the Spring et al. reference wherein a yield of 75% is obtained.

The present invention provides a novel method for making an N-alkylphthalimide which is based on the reaction of phthalic anhydride with an alkyl amine in two separate reaction zones that are operated at two different temperatures. This method is capable of producing yields in excess of 99.5% of N-alkylphthalimide.

Accordingly, it is a primary object of this invention to provide an improved method for making N-alkylphthalimides that gives a pure product in high yields.

BRIEF DESCRIPTION OF THE DRAWINGS

Fig. 1 is a schematic of a pilot plant apparatus for the practice of the inventive process.

Fig. 2 is a schematic of a large scale apparatus for the practice of the invention.

## DETAILED DESCRIPTION OF THE INVENTION

According to the present invention, there is provided a method for the synthesis of an N-alkylphthalimide which comprises:

(a) contacting molten phthalic anhydride with a stoichiometric excess of an alkylamine to form a reaction mixture in a first reaction zone provided with agitation means;

(b) transferring said reaction mixture to a second reaction zone maintained at a higher temperature than said first reaction zone;

(c) adding additional alkylamine to said second reaction zone; and

(d) recovering substantially pure N-alkylphthalimide from said second reaction zone.

The preferred N-alkylphthalimide is N-methylphthalimide which is derived from monomethylamine and phthalic anhydride.

Other analogous N-alkylphthalimides may be derived using ethylamine; n-propyl amine; n-butylamine and the like.

The first reaction zone is preferably a continuously stirred tank reactor which may be operated with a recycle loop that functions as the second reaction zone or with a second continuously stirred tank reactor that functions as the second reaction zone.

The second reaction zone is always operated at a temperature higher than the first reaction zone.

Agitation may be supplied to the reaction zones by any conventional stirring, shaking or turbulent flow techniques such as the use of a stream of inert gas bubbles.

The phthalic anhydride is melted and fed to the first reaction zone which is maintained at a temperature of from about 155- 195°C; and a pressure of 15-60 psia (pounds per square inch absolute) and preferably 35-60 psia. The residence time is from 1 to 2 hours.

The output of the first reaction zone is separated into gas and liquid phases with the gas being directed to waste treatment and the liquid being directed to the second reaction zone. The liquid in the second reaction zone is admixed with an additional 20-50% molar excess of the alkyl amine. The temperature in the second reaction zone is from 175-200°C; preferably from 180-195°C; and at a pressure of 15-100 psia, preferably 35-60 psia. The residence time in the second reaction zone is from 0.5 to 2 hours. The output of the second reaction zone is separated into a gaseous stream and a liquid product stream which is substantially pure N-alkylphthalimide.

The use of the alkylamine in more than about 5% in excess of the stoichiometric amount in the first reacton zone has little effect on the conversion rate. Doubling the residence time or tripling the agitation rate increases the conversion by about 0.2%. Pressure and temperature increase also have little effect on conversion. The use of a second reaction zone increases the conversion to more than 99.9% with very little sensitivity to process variables.

A stainless steel reactor may be used provided that the temperature is kept above 140°C.

The reactor should be capable of controlling the heat of reaction and usually it is necessary to supply an internal or external cooling means. The temperature in the second reaction zone is critical to the optimum performance of the process. If temperatures of 145-155°C are used in the second reaction zone, it has been found that the N,N'-dialkylamide may comprise from 5 to 6% of the product.

## DESCRIPTION OF THE PREFERRED EMBODIMENTS

The following embodiments illustrate the process of this invention. They are merely illustrative and are not to be construed to limit the invention in any manner whatsoever.

## EXAMPLES 1-16

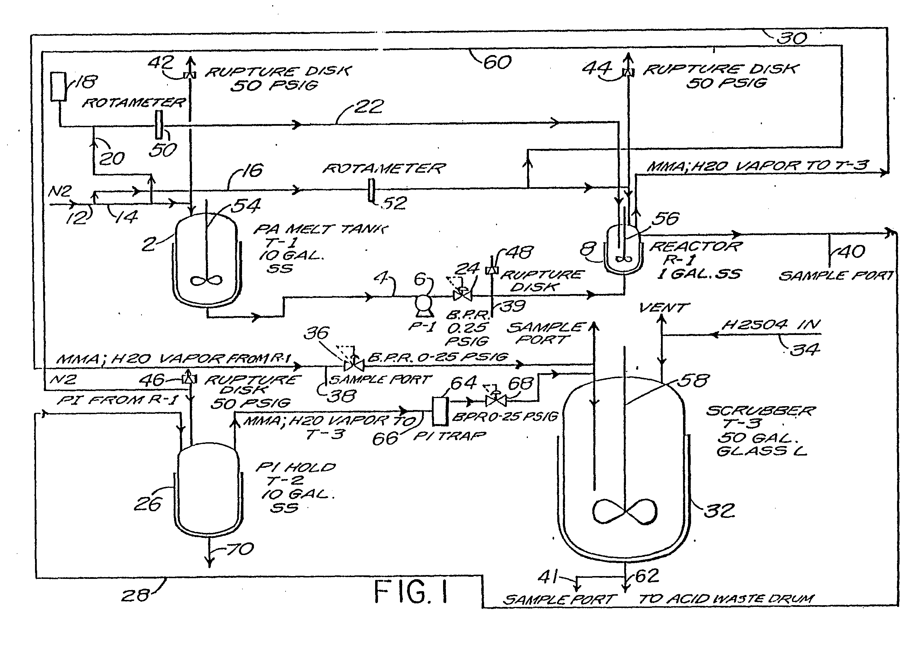

A pilot plant apparatus arranged in accordance with Fig. 1 is used to make N-methylphthalimide. Phthalic anhydride is melted in melt tank 2 and is then pumped through line 4 by pump 6 to reactor 8. Nitrogen is supplied from line 12 which is connected to PA melt tank 2 by line 14 and to reactor 8 through line 16. Monomethylamine is fed from cylinder 18 through line 22 to reactor 8. Valve 68 is used to control pressure in reactor 8. The liquid product of reactor 8 is passed to hold tank 26 through line 28. The vapor fraction from reactor 8 is sent by line 30 to scrubber 32 which is provided with $H_2SO_4$ through line 34 to neutralize any monomethylamine. Valve 36 is used to control the flow in line 30. Sample ports 38, 39, 40 and 41 are used to monitor the composition of the materials flowing in lines 30 and 28. Rupture disks 42, 44, 46 and 48 are conventional safety devices to relieve any overpressure in the system. Waste line 62 is connected to a waste disposal receptacle. Trap 64 is used to isolate any N-methylphthalimide from waste recovery line 66 prior to passing the waste through control valve 68. N-methylphthalimide is removed from line 70.

Typically, 5.4 lb/hour flow of phthalic anhydride in conjunction with a 10% molar excess of monomethylamine in phthalic anhydride is established through the first step of an apparatus of the type described in Fig. 1. Reactor 8 temperature is 189°C, the agitator speed is 820 rpm, and the pressure is 35 psia. The phthalic anhydride content of the outflow is 0.4%. The conditions employed and the results obtained in twelve first stage reactor runs are summarized in Table 1, the typical run above-described being listed as Example 12, entry 3, Table 1.

A run through a second stage of reaction was simulated by collecting material from PI hold tank 26 and remelting it in melt tank 22. Typically, a run was made with a feed of 10 lb/hr of a mixture of N-methylphthalimide containing 8% phthalic anhydride with about a 20% excess of monomethylamine. The reactor temperature was 175°C, agitator speed was 800 rpm, and the pressure was 16 psia. The outlet stream contained 0.05% phthalic anhydride and 0.4% of N,N-dimethylphthalimide. The results from a series of such runs are set forth in Table 2, the typical run being Example 16, entry 1.

TABLE 1.    REACTION BETWEEN PHTHALIC ANHYDRIDE (PA)
AND MONOMETHYLAMINE (MMA) GENERAL

| Example | PA Flow lb/hr | MMA % Excess | Reactor Temp.,°C. | Agitator RPM | Pressure psia | Outlet PA % |
|---------|------|---------|------|------|----|---------|
| 1 - | 4.8 | ∿20 | 158 | | 15 | 0.5 |
| 2 | 4.8 | ∿ 0 | 160 | | 15 | 1.1 |
| | 4.8 | ∿10 | 161 | | 15 | .4-.5 |
| | 4.8 | ∿20 | 161 | | 15 | .4-.5 |
| 3 | 4.8 | ∿-5 | 161 | | 15 | 2.5 |
| | 4.8 | ∿ 0 | 161 | | 15 | .8-.9 |
| | 4.8 | ∿ 5 | 161 | | 15 | .6-.7 |
| | 4.8 | ∿10 | 161 | | 15 | .5 |
| 4 | 4.8 | ∿10 | 158 | | 15 | .5 |
| | 3.6 | ∿10 | 154 | | 15 | .3-.5 |
| | 3.6 | ∿10 | 154 | | 15 | .2-.4 |
| 5 | 4.8 | ∿20 | 156 | 1000 | 15 | .3 |
| | 4.8 | ∿20 | 156 | 500 | 15 | .3-.7 |
| | 4.8 | ∿20 | 156 | 1500 | 15 | .1-.5 |
| 6 | 18.5* | ∿10 | 173 | 600 | 15 | .7-1.0 |
| 7 | 9.7 | 0** | 164 | 800 | 24 | 2-5 |
| | 9.7 | 0 | 164 | 0 | 24 | 3-4 |
| 8 | 7.8 | 7.5 | 176 | 1150 | 25 | .6 |
| | 7.8 | 15 | 176 | 1150 | 25 | .3-.5 |
| 9 | 5.4 | 0 | 162 | 840 | 15 | .74 |
| | 5.4 | 5 | 162 | 840 | 15 | .46 |
| | 5.4 | 10 | 162 | 840 | 15 | .40 |
| 10 | 9.6 | 0 | 191 | 840 | 15 | 2.4 |
| | 9.6 | 5 | 191 | 840 | 15 | .7 |
| | 9.6 | 10 | 191 | 840 | 15 | .6 |
| 11 | 9.6 | 0 | 162 | 800 | 35 | 1.1 |
| | 9.6 | 5 | 162 | 800 | 35 | .6 |
| | 9.6 | 10 | 162 | 800 | 35 | .6 |
| 12 | 5.4 | 0 | 189 | 820 | 35 | .8 |
| | 5.4 | 5 | 190 | 820 | 35 | .5 |
| | 5.4 | 10 | 189 | 820 | 35 | .4 |
| | 5.4 | 0 | 191 | 0 | 0 | 1.4 |

*   91% PA, 9% phthalimide (PI)
**  Mass balance run

TABLE 2. REACTION BETWEEN PHTHALIC ANHYDRIDE (PA) AND
MONOMETHYLAMINE (MMA). SECOND STAGE REACTOR RUNS

| Example | Feed Flow lb/hr | PA in Feed % | MMA Excess % | Reactor Temp. °C. | Agitator RPM | Pressure psia % | Outlet PA % | Diamide % |
|---------|-----------------|--------------|--------------|-------------------|--------------|-----------------|-------------|-----------|
| 13 | 4.8 | 17 | 50 . | 151 | 1000 | 15 | .06 | 2.2 |
|  | 9.6 | 17 | 50 | 152 | 1000 | 15 | .10 | 1.6 |
|  | 15 | 17 | 50 | 154 | 1000 | 15 | .15 | 1.0 |
|  | 20 | 17 | 50 | 157 | 1000 | 15 | .16 | .15 |
| 14 | 25 | 8 | 150 | 150 | 530 | 15 | .15-.30 | 1.9 |
|  | 25 | 8 | 150 | 150 | 0 | 15 | .25 | 1.5 |
|  |  | PRODUCT TANK COMPOSITE | | | | | .015 | 2.5 |
| 15 | 9.6 | 0.2 | | 145 | 520 | 15 | .10 | |
|  | 17 | 0.2 | | 146 | 500 | 15 | .06 | 4.4 |
|  |  | PRODUCT TANK COMPOSITE | | | | | .06 | 4.3 |
| 16 | 10 | 8 | 20 | 175 | 800 | 30 | .05 | .4 |
|  | 10 | 8 | 50 | 176 | 830 | 30 | .05 | .3 |
|  |  | PRODUCT TANK COMPOSITE | | | | | .015 | 1.1 |
|  |  | PRODUCT TANK COMPOSITE | | | | | .1 | .1-.2 |

- 8 -

EXAMPLE 17

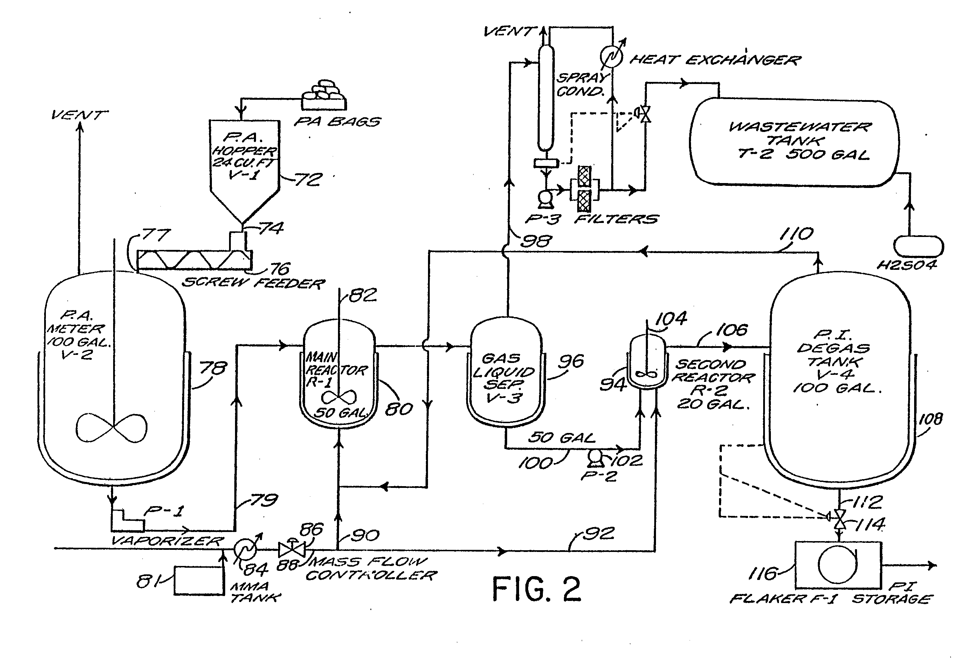

A commercial type apparatus for the production of N-methylphthalimide is described in Fig. 2  Phthalic anhydride is fed into hopper 72 from which it passes through line 74 to screw feeder 76 and from these into line 77 which is connected to melter 78. Melter 78 is connected by line 79 to the main reactor 80 which is provided with stirrer 82. Monomethylamine is fed into vaporizer 84 from tank 81 through valve 86 into line 88. Line 88 divides in line 90 and line 92 connected, respectively, to main reactor 80 and second reactor 94. The output of reactor 80 is fed to gas-liquid separator 96 where the gaseous fraction is sent via line 98 to a waste treatment loop. The liquid phase is fed via line 100 by pump 102 to second reactor 94 where it is agitated by a stirrer 104. The output of second reactor 94 is fed to degassing tank 108 via line 106. The gas fraction from degassing tank 108 is recycled to main reactor 80 via line 110. The product is transferred from degassing tank 108 by line 112 controlled by valve 114 to flaker 50 in which is collected substantially pure N-methylphthalimide.

The products of the present process and its analogs are pure enough to be nitrated to 4-nitro-N-methylphthalimide, (4-NPI) and its analogs. 4-NPI is used in making commercially important resins. The results above demonstrate that the direct reaction of molten PA and gaseous MMA in a single-stage continuous stirred tank reactor produces 99% PI over a wide range of temperatures, pressures, residence times, and agitation rates. About 8% excess MMA is required to achieve 99.4% PA conversion in one pass. Greater excess of MMA does not increase conversion significantly. A second pass through a continuous stirred tank reactor with a moderate excess of MMA increases PA conversion to 99.9+%. Operations of the second stage reactor at 175°C. reduces the content of the most significant organic impurity, N,N'-dimethylphthalimide to less than 0.5%. Other organic impurities were very minor, totaling less than 0.3%.

The above-mentioned patents and publications are incorporated herein by reference.

Many variations in the instant invention will suggest themselves to those skilled in this art in light of the above, detailed description. All such obvious modifications are within the full intended scope of the appended claims.

0184595

CLAIMS

1. A method for the synthesis of an N-alkylphthalimide which comprises:

   (a) contacting molten phthalic anhydride with a stoichiometric excess of an alkyl amine to form a reaction mixture in a first reaction zone that is provided with agitation means;

   (b) transferring said reaction mixture to a second reaction zone that is maintained at a higher temperature than said first reaction zone;

   (c) adding additional alkylamine to said second reaction zone; and

   (d) recovering substantially pure N-alkylphthalimide from said second reaction zone.

2. A method for the synthesis of an N-alkylphthalimide as defined in Claim 1 wherein the N-alkylphthalimide is N-methylphthalimide and the first and second reaction zones are separate continuously stirred tank reactors.

3. A method for the synthesis of N-methylphthalimide as defined in Claim 2 wherein the first reaction zone is maintained at a temperature of 155-195°C and the second reaction zone is maintained at a temperature of 175-200°C.

4. A method for the synthesis of N-methylphthalimide as defined in Claim 3 wherein the output of the first reaction zone is divided into a gas and liquid phase and the liquid gas is passed to the second reaction zone and the gas phase is passed to waste treatment.

5. A method for the synthesis of N-methylphthalimide as defined in Claim 3 wherein the process is carried out at 15-60 psia pressure in the first reaction zone and 15-100 psia in the second.

FIG. 1

FIG. 2

VENT

PA BAGS

P.A. HOPPER 24 CU.FT V-1 — 72

VENT

HEAT EXCHANGER

SPRAY COND.

WASTEWATER TANK T-2 500 GAL.

P-3 FILTERS

98

110

H2SO4

77

74

76

SCREW FEEDER

P.A. METER 100 GAL. V-2

78

82

104

106

P.I. DEGAS TANK V-4 100 GAL.

MAIN REACTOR R-1 50 GAL.

80

GAS LIQUID SEP. V-3

96

94

SECOND REACTOR R-2 20 GAL.

108

50 GAL

100

P-2

102

92

79

P-1

VAPORIZER

84

86

88

90

MASS FLOW CONTROLLER

81

MMA TANK

112

114

116

FLAKER F-1 STORAGE

PI

2/2

0184595

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | "Methoden der organischen Chemie" (Houben-Weyl), 4th edition, vol. XI/2, Georg Thieme Verlag, Stuttgart, DE, 1958, pages 16-19, F. MÖLLER: "Umwandlung von primären und sekundären Aminen" * Pages 16-19 * | 1-5 | C 07 D 209/48 |
| D,A | JOURNAL OF THE CHEMICAL SOCIETY, 1945, pages 625-628, The Chemical Society, London, GB; F.S. SPRING et al.: "The application of the Hofmann reaction to the synthesis of heterocyclic compounds. Part III. Synthesis of 3-alkyl-2 : 4-diketo-1 : 2 : 3 : 4-tetrahydro-quinazolines from N-alkylphthalamides" | 1-5 | |
| A | GB-A-1 484 243 (B.V. DRAKE) * Whole document * | 1-5 | TECHNICAL FIELDS SEARCHED (Int. Cl.4) C 07 D 209/00 |
| D,A | DE-A-2 628 783 (GENERAL ELECTRIC CO.) * Whole document * | 1-5 | |
| A | DE-A-2 412 466 (GENERAL ELECTRIC CO.) * Whole document * & US - A - 3 879 428 (Cat. D,A) | 1-5 | |

The present search report has been drawn up for all claims

| Place of search | Date of completion of the search | Examiner |
|---|---|---|
| THE HAGUE | 28-08-1985 | MAISONNEUVE J.A. |